# EUROPEAN PATENT APPLICATION

(11) **EP 1 247 816 A1**
(43) Date of publication of application: **09.10.2002**
(21) Application number: 00980069.9
(22) Date of filing: 28.11.2000
(51) Int. Cl.: C07K 14/435, C12N 15/12, C12N 15/63, C12N 1/21, A61K 38/17, A61K 39/00, C07K 16/18

(54) **IMMUNOGEN, ANTIVENOM AND VACCINE AGAINST THE VENOM OF THE BLACK WIDOW SPIDER**

(30) Priority: 03.12.1999 MX 9911191
(71) Applicant: UNIVERSIDAD NACIONAL AUTONOMA DE MEXICO, Mexico 04510 D.F. (MX); Shemyakin and Ovchinnikov Institute of Bioorganic Chemistry, Moscou V-437 (RU)
(72) Inventor: GURROLA BRIONES, Georgina, Cuernavaca, Morelos 62230 (MX); ALAGON CANO, Alejandro, Cuernavaca, Morelos 62240 (MX); POSSANI POSTAY, Lourival Domingos, Cuernavaca, Morelos 62290 (MX); GRISHIN, Eugene Vasilevich, Moscow 125284 (RU); LIPKIN, Alexei Valerevich, Moscow 121359 (RU); VOLYNSKI, Kirill Evgenevich, Zhukov 249031 Kaluzhskaya (RU)
(74) Representative: Chapman, Paul William
(86) International application number: MX0000048
(87) International publication number: WO01040290

(57) **Abstract**

The present invention refers to a recombinant polypeptide that comprises the N-terminal region of α-Latrotoxin and that is capable of generating an immune response in mammals that is effective in neutralizing the whole venom of the black widow spider. Similarly, it refers to the cDNA that encodes it, the vector for its expression, the bacterial cells transformed with said vector and the method for producing the polypeptide by means of the culture of said cells. In another embodiment, the present invention also refers to the use of the recombinant polypeptide as a vaccine against the venom of the black widow spider and the pharmaceutically acceptable preparations of the vaccine. Similarly, in another embodiment, the present invention refers to the use of the recombinant polypeptide as immunogen for the generation of sera against the venom of the black widow spider, obtaining the serotherapeutic agents through purification of the serum and obtaining fabotherapeutic agents through enzymatic hydrolysis of the serum antibodies. Furthermore, in another embodiment, the present invention refers to an immunogenic matrix useful in the separation and purification of antibodies and their fragments specific against the venom of the black widow spider.

SEQUENCE LISTING

<110> UNIVERSIDAD NACIONAL AUTÓNOMA DE MÉXICO

SHEMYAKIN AND OVCHINNIKOV INSTITUTE OF BIOORGANIC CHEMISTRY
Possani Postay, Lourival D

<120> INMUNOGEN, ANTI-VENOM AND VACCINE AGAINST THE VENOM OF THE
BLACK WIDOW SPIDER

<130> a-Latrotoxina

<140>
<141>

<150> MX/9911191
<151> 1999-12-03

<160> 7

<170> PatentIn Ver. 2.1

<210> 1
<211> 482
<212> PRT
<213> Latrodectus mactans

<220>
<221> PEPTIDE
<222> (1)..(482)

<220>
<221> SITE
<222> (13)..(468)
<223> First 456 amino acids of the alfa-Latrotoxin

<220>
<221> SITE
<222> (1)..(12)
<223> sequence derived from the vector, compric a poly
His for purification

<300>
<301> Kiyatkin, N. I.
Duluvoba, I. E.
Chekhovskaya, I. A.
Grishin, Eugene V.
<302> Cloning and structure of cDNA encoding a-Latrotoxin
from black widow spider venom
<303> FEBS Lett.
<304> 270
<305> 1,2
<306> 127-131
<307> 1990-09

<313> 13 TO 468

<400> 1
Met Ala Arg Ile His His His His His His Gly Ser Glu Gly Glu Asp
1               5                   10                  15

Leu Thr Leu Glu Glu Lys Ala Glu Ile Cys Ser Glu Leu Glu Leu Gln
            20                  25                  30

Gln Lys Tyr Val Asp Ile Ala Ser Asn Ile Ile Gly Asp Leu Ser Ser
        35                  40                  45

Leu Pro Ile Val Gly Lys Ile Ala Gly Thr Ile Ala Ala Ala Ala Met
    50                  55                  60

Thr Ala Thr His Val Ala Ser Gly Arg Leu Asp Ile Glu Gln Thr Leu
65                  70                  75                  80

Leu Gly Cys Ser Asp Leu Pro Phe Asp Gln Ile Lys Glu Val Leu Glu
                85                  90                  95

Asn Arg Phe Asn Glu Ile Asp Arg Lys Leu Asp Ser His Ser Ala Ala
            100                 105                 110

Leu Glu Glu Ile Thr Lys Leu Val Glu Lys Ser Ile Ser Val Val Glu
        115                 120                 125

Lys Thr Arg Lys Gln Met Asn Lys Arg Phe Asp Lys Val Met Lys Ser
    130                 135                 140

Ile Gln Asp Ala Lys Val Ser Pro Ile Val Ser Lys Ile Asn Asn Phe
145                 150                 155                 160

Ala Arg Tyr Phe Asp Thr Glu Lys Glu Arg Ile Arg Gly Leu Lys Leu
                165                 170                 175

Asn Asp Tyr Ile Leu Lys Leu Glu Glu Pro Asn Gly Ile Leu Leu His
            180                 185                 190

Leu Lys Glu Ser Arg Thr Pro Thr Asp Asp Ser Leu Gln Ala Pro Leu
        195                 200                 205

Phe Ser Ile Ile Glu Glu Gly Tyr Ala Val Pro Lys Ser Ile Asp Asp
    210                 215                 220

Glu Leu Ala Phe Lys Val Leu Tyr Ala Leu Leu Tyr Gly Thr Gln Thr
225                 230                 235                 240

Tyr Val Ser Val Met Phe Phe Leu Leu Glu Gln Tyr Ser Phe Leu Ala
                245                 250                 255

Asn His Tyr Tyr Glu Lys Gly Tyr Leu Glu Lys Tyr Asp Glu Tyr Phe
            260                 265                 270

Asn Ser Leu Asn Asn Val Phe Leu Asp Phe Lys Ser Ser Leu Val Gly
        275                 280                 285

Thr Gly Thr Ser Asn Asn Glu Gly Leu Leu Asp Arg Val Leu Gln Val
    290                 295                 300

Leu Met Thr Val Lys Asn Ser Glu Phe Leu Gly Leu Glu Lys Asn Gly
305                 310                 315                 320

Val Asp Glu Met Leu Asn Glu Lys Ile Asn Leu Phe Asn Lys Ile Lys
                325                 330                 335

Glu Glu Ile Glu Gly Lys Gln Lys Met Thr Leu Ser Glu Thr Pro Glu
            340                 345                 350

Asn Phe Ala Gln Ile Ser Phe Asp Lys Asp Ile Thr Thr Pro Ile Gly
        355                 360                 365

Asp Trp Arg Asp Gly Arg Glu Val Arg Tyr Ala Val Gln Tyr Ala Ser
    370                 375                 380

Glu Thr Leu Phe Ser Lys Ile Ser His Trp Ser Asp Pro Val Ser Val
385                 390                 395                 400

Arg Glu Lys Ala Cys Pro Thr Leu Arg Met Pro Val Asp Gln Thr Arg
                405                 410                 415

Arg Asn Val Leu Val Phe Arg Lys Phe Asp Ser Ser Lys Pro Gln Leu
            420                 425                 430

Val Gly Glu Ile Thr Pro Tyr Leu Ser Asn Phe Ile Asp Ile Asp Arg
        435                 440                 445

Asp Leu Tyr Asn Ala Ala Ser Asn Pro Asp Ser Ala Val Gly Phe Lys
    450                 455                 460

Glu Phe Thr Lys Val Asp Leu Gln Pro Lys Leu Ile Asp Asp Asn Cys
465                 470                 475                 480

Gln Thr

<210> 2
<211> 1449
<212> ADN
<213> Latrodectus mactans

<220>
<221> CDS
<222> (1)..(1449)

<220>
<221> mat_peptide
<222> (1)..(1446)
<223> Codifies a polipeptide that comprices the first
      456 amino acids from the alfa-Latrotoxina

<300>
<301> Kiyatkin, N. I.
      Dulubova, I. E.
      Chekhovskaya, I. A.
      Grishin, Eugene V.
<302> Cloning ans structure of cDNA encoding a-Latrtoxin from
      black widow spider venom
<303> FEBS Lett.
<304> 270
<305> 1,2
<306> 127-131
<307> 1990-09
<313> 37 TO 1404

<400> 2
atg gct aga att cat cac cat cac cat cac gga tcc gaa gga gaa gat      48
Met Ala Arg Ile His His His His His His Gly Ser Glu Gly Glu Asp
1               5                   10                  15

tta act tta gag gaa aaa gca gaa ata tgc tca gaa tta gaa ctc caa      96
Leu Thr Leu Glu Glu Lys Ala Glu Ile Cys Ser Glu Leu Glu Leu Gln
            20                  25                  30

caa aaa tat gta gat ata gct tcc aat att att ggc gat ctt tcc tct      144
Gln Lys Tyr Val Asp Ile Ala Ser Asn Ile Ile Gly Asp Leu Ser Ser
        35                  40                  45

ctt cca ata gtt ggt aaa att gct ggc acc ata gct gca gct gcg atg      192
Leu Pro Ile Val Gly Lys Ile Ala Gly Thr Ile Ala Ala Ala Ala Met
    50                  55                  60

acc gct acc cat gtt gct tct ggg agg tta gat atc gag cag acg ttg      240
Thr Ala Thr His Val Ala Ser Gly Arg Leu Asp Ile Glu Gln Thr Leu
65                  70                  75                  80

ttg ggt tgc agt gac tta cct ttc gat cag att aag gaa gtt ctt gaa      288
Leu Gly Cys Ser Asp Leu Pro Phe Asp Gln Ile Lys Glu Val Leu Glu
                85                  90                  95

aat agg ttc aac gaa ata gac agg aag ttg gat agt cat agt gcg gcg      336
Asn Arg Phe Asn Glu Ile Asp Arg Lys Leu Asp Ser His Ser Ala Ala
            100                 105                 110

ctt gag gaa att act aaa ctg gtt gag aaa agt ata tcc gtg gta gaa      384
Leu Glu Glu Ile Thr Lys Leu Val Glu Lys Ser Ile Ser Val Val Glu
        115                 120                 125

aaa aca cgg aag cag atg aat aag agg ttt gac aaa gtc atg aag tct 432
Lys Thr Arg Lys Gln Met Asn Lys Arg Phe Asp Lys Val Met Lys Ser
    130                 135                 140

att cag gac gca aaa gtt tcc cca att gtt tct aaa atc aat aat ttt 480
Ile Gln Asp Ala Lys Val Ser Pro Ile Val Ser Lys Ile Asn Asn Phe
145                 150                 155                 160

gca aga tat ttt gat act gaa aag gaa aga atc cgt ggt tta aag tta 528
Ala Arg Tyr Phe Asp Thr Glu Lys Glu Arg Ile Arg Gly Leu Lys Leu
                165                 170                 175

aat gat tat att ctt aag ctt gaa gaa cca aat ggc ata cta ctt cat 576
Asn Asp Tyr Ile Leu Lys Leu Glu Glu Pro Asn Gly Ile Leu Leu His
            180                 185                 190

ttg aaa gag tcg cga act cca acg gat gat agt tta caa gct cct tta 624
Leu Lys Glu Ser Arg Thr Pro Thr Asp Asp Ser Leu Gln Ala Pro Leu
        195                 200                 205

ttc agt att att gaa gaa gga tat gca gta ccc aaa tct att gat gac 672
Phe Ser Ile Ile Glu Glu Gly Tyr Ala Val Pro Lys Ser Ile Asp Asp
    210                 215                 220

gaa tta gct ttt aaa gtt ctc tat gca ttg ctg tat ggt acc cag aca 720
Glu Leu Ala Phe Lys Val Leu Tyr Ala Leu Leu Tyr Gly Thr Gln Thr
225                 230                 235                 240

tat gtc tcg gtt atg ttt ttt ctc cta gaa caa tac tct ttc ctg gct 768
Tyr Val Ser Val Met Phe Phe Leu Leu Glu Gln Tyr Ser Phe Leu Ala
                245                 250                 255

aac cat tat tac gag aaa gga tat tta gaa aag tat gat gaa tac ttt 816
Asn His Tyr Tyr Glu Lys Gly Tyr Leu Glu Lys Tyr Asp Glu Tyr Phe
            260                 265                 270

aat agc ctt aat aat gta ttt tta gat ttt aaa tcg tca ctt gtg ggt 864
Asn Ser Leu Asn Asn Val Phe Leu Asp Phe Lys Ser Ser Leu Val Gly
        275                 280                 285

acc ggc aca tct aat aat gaa ggt ctt ctt gat aga gtg ctt caa gtt 912
Thr Gly Thr Ser Asn Asn Glu Gly Leu Leu Asp Arg Val Leu Gln Val
    290                 295                 300

tta atg act gtt aag aat agc gag ttc tta gga ctt gaa aaa aat ggt 960
Leu Met Thr Val Lys Asn Ser Glu Phe Leu Gly Leu Glu Lys Asn Gly
305                 310                 315                 320

gta gat gaa atg ctg aac gag aaa ata aat tta ttt aat aaa ata aag 1008
Val Asp Glu Met Leu Asn Glu Lys Ile Asn Leu Phe Asn Lys Ile Lys
                325                 330                 335

gaa gaa atc gaa ggc aag caa aaa atg aca ttg agt gag aca cca gaa 1056
Glu Glu Ile Glu Gly Lys Gln Lys Met Thr Leu Ser Glu Thr Pro Glu

            340                 345                 350

aat ttt gcg caa att tca ttt gat aaa gat ata aca act ccc atc gga 1104
Asn Phe Ala Gln Ile Ser Phe Asp Lys Asp Ile Thr Thr Pro Ile Gly
        355                 360                 365

gat tgg aga gat ggt cgg gaa gta cgt tat gct gta caa tac gca agt 1152
Asp Trp Arg Asp Gly Arg Glu Val Arg Tyr Ala Val Gln Tyr Ala Ser
    370                 375                 380

gaa acc tta ttt tct aaa atc agt cat tgg tct gat ccc gtg agt gtg 1200
Glu Thr Leu Phe Ser Lys Ile Ser His Trp Ser Asp Pro Val Ser Val
385                 390                 395                 400

aga gag aaa gca tgt cct act ctc aga atg cca gtg gac caa act cga 1248
Arg Glu Lys Ala Cys Pro Thr Leu Arg Met Pro Val Asp Gln Thr Arg
                405                 410                 415

aga aac gtg ctt gtt ttc aga aaa ttt gac agc agc aaa ccc caa ctg 1296
Arg Asn Val Leu Val Phe Arg Lys Phe Asp Ser Ser Lys Pro Gln Leu
            420                 425                 430

gtt ggc gaa atc aca cca tac cta tca aat ttc atc gac att gat aga 1344
Val Gly Glu Ile Thr Pro Tyr Leu Ser Asn Phe Ile Asp Ile Asp Arg
        435                 440                 445

gat ctt tat aat gcc gct agt aat cct gat tca gct gta ggt ttt aag 1392
Asp Leu Tyr Asn Ala Ala Ser Asn Pro Asp Ser Ala Val Gly Phe Lys
    450                 455                 460

gaa ttc acg aaa gtc gac ctg cag ccc aag ctt atc gat gat aac tgt 1440
Glu Phe Thr Lys Val Asp Leu Gln Pro Lys Leu Ile Asp Asp Asn Cys
465                 470                 475                 480

caa aca tga 1449
Gln Thr

<210> 3
<211> 482
<212> PRT
<213> Latrodectus mactans

<400> 3
Met Ala Arg Ile His His His His His His Gly Ser Glu Gly Glu Asp
1               5                   10                  15

Leu Thr Leu Glu Glu Lys Ala Glu Ile Cys Ser Glu Leu Glu Leu Gln
            20                  25                  30

Gln Lys Tyr Val Asp Ile Ala Ser Asn Ile Ile Gly Asp Leu Ser Ser
        35                  40                  45

Leu Pro Ile Val Gly Lys Ile Ala Gly Thr Ile Ala Ala Ala Ala Met
    50                  55                  60

Thr Ala Thr His Val Ala Ser Gly Arg Leu Asp Ile Glu Gln Thr Leu
65                  70                  75                  80

Leu Gly Cys Ser Asp Leu Pro Phe Asp Gln Ile Lys Glu Val Leu Glu
                85                  90                  95

Asn Arg Phe Asn Glu Ile Asp Arg Lys Leu Asp Ser His Ser Ala Ala
            100                 105                 110

Leu Glu Glu Ile Thr Lys Leu Val Glu Lys Ser Ile Ser Val Val Glu
        115                 120                 125

Lys Thr Arg Lys Gln Met Asn Lys Arg Phe Asp Lys Val Met Lys Ser
    130                 135                 140

Ile Gln Asp Ala Lys Val Ser Pro Ile Val Ser Lys Ile Asn Asn Phe
145                 150                 155                 160

Ala Arg Tyr Phe Asp Thr Glu Lys Glu Arg Ile Arg Gly Leu Lys Leu
                165                 170                 175
Asn Asp Tyr Ile Leu Lys Leu Glu Glu Pro Asn Gly Ile Leu Leu His
            180                 185                 190

Leu Lys Glu Ser Arg Thr Pro Thr Asp Asp Ser Leu Gln Ala Pro Leu
        195                 200                 205

Phe Ser Ile Ile Glu Glu Gly Tyr Ala Val Pro Lys Ser Ile Asp Asp
    210                 215                 220

Glu Leu Ala Phe Lys Val Leu Tyr Ala Leu Leu Tyr Gly Thr Gln Thr
225                 230                 235                 240

Tyr Val Ser Val Met Phe Phe Leu Leu Glu Gln Tyr Ser Phe Leu Ala
                245                 250                 255

Asn His Tyr Tyr Glu Lys Gly Tyr Leu Glu Lys Tyr Asp Glu Tyr Phe
            260                 265                 270

Asn Ser Leu Asn Asn Val Phe Leu Asp Phe Lys Ser Ser Leu Val Gly
        275                 280                 285

Thr Gly Thr Ser Asn Asn Glu Gly Leu Leu Asp Arg Val Leu Gln Val
    290                 295                 300

Leu Met Thr Val Lys Asn Ser Glu Phe Leu Gly Leu Glu Lys Asn Gly
305                 310                 315                 320

Val Asp Glu Met Leu Asn Glu Lys Ile Asn Leu Phe Asn Lys Ile Lys
                325                 330                 335

Glu Glu Ile Glu Gly Lys Gln Lys Met Thr Leu Ser Glu Thr Pro Glu
            340                 345                 350

Asn Phe Ala Gln Ile Ser Phe Asp Lys Asp Ile Thr Thr Pro Ile Gly
        355                 360                 365

Asp Trp Arg Asp Gly Arg Glu Val Arg Tyr Ala Val Gln Tyr Ala Ser
    370                 375                 380

Glu Thr Leu Phe Ser Lys Ile Ser His Trp Ser Asp Pro Val Ser Val
385                 390                 395                 400

Arg Glu Lys Ala Cys Pro Thr Leu Arg Met Pro Val Asp Gln Thr Arg
                405                 410                 415

Arg Asn Val Leu Val Phe Arg Lys Phe Asp Ser Ser Lys Pro Gln Leu
            420                 425                 430

Val Gly Glu Ile Thr Pro Tyr Leu Ser Asn Phe Ile Asp Ile Asp Arg
        435                 440                 445

Asp Leu Tyr Asn Ala Ala Ser Asn Pro Asp Ser Ala Val Gly Phe Lys
    450                 455                 460

Glu Phe Thr Lys Val Asp Leu Gln Pro Lys Leu Ile Asp Asp Asn Cys
465                 470                 475                 480

Gln Thr

<210> 4
<211> 27
<212> ADN
<213> Artificial Sequence

<220>
<221> primer_bind
<222> (1)..(27)

<220>
<221> CDS
<222> (10)..(27)
<223> Codifies the first 6 amino ácidos of the
      alfa-Latrotoxina
<220>
<223> Description of the Artificial Sequence: Primer
      AL-1

<400> 4
ttgggatcc gaa gga gaa gat tta act                                27
          Glu Gly Glu Asp Leu Thr
            1               5
<210> 5

<211> 6
<212> PRT
<213> Artificial Sequence
<223> Description of the Artificial Sequence: Primer
      AL-1

<400> 5
Glu Gly Glu Asp Leu Thr
  1               5


<210> 6
<211> 29
<212> ADN
<213> Artificial Sequence

<220>
<221> primer_bind
<222> (1)..(29)

<220>
<221> CDS
<222> (9)..(29)
<223> Codifies amino acids 451 a 456 of the
      alfa-Latrotoxina

<220>
<223> Description of the Artificial Sequence: Prmer
      AL-2R


<400> 6
gaaccgtc gac ttt cgt gaa ttc ctt aaa                                29
         Asp Phe Arg Glu Phe Leu Lys
           1               5


<210> 7
<211> 7
<212> PRT
<213> Artifitial Sequence
<223> Description of the Artificial Sequence: Primer
      AL-2R

<400> 7
Asp Phe Arg Glu Phe Leu Lys
  1               5

## Description

### TECHNICAL FIELD

The present invention refers to a polypeptide that consists of a fragment of the toxin α-Latrotoxin from the venom of the black widow spider, which, when applied to a mammal, stimulates an immune response that is effective in neutralizing the toxic action of the whole arachnid venom. It also refers to the use of this polypeptide as a vaccine in mammals and as an immunogen for the production of antibodies in mammals against the whole arachnid venom; it also refers to the serotherapeutic and fabotherapeutic agents obtained from said antibodies and an immunogenic matrix capable of specifically binding neutralizing antibodies of the arachnid venom. Aanother inbodiment of the present invention also includes a DNA fragment that codes for the polypeptide and the expression construction for said fragment, as well as the bacterial cells transformed with said construction and the method to produce the polypeptide in a recombinant way.

### BACKGROUND

A toxin is a protein or peptide that has harmful effects in man or in animals. Venoms normally consist of a large set of toxins; they are a relatively complex mixture of toxins that can cause morbidity or a significant degree of mortality in humans and animals. Many animals like reptiles, mollusks and arachnids produce this type of venom that they use as defense mechanisms and/or as mechanisms to kill their prey. Their use has even been reported as self-sprinkling by scorpions, as a disinfectant since there are antibiotic peptides in their venom (Mexican patent application No. 985,522). For this reason, toxins are usually present that are specific against insects, crustaceans and mammals.
Among the best known venomous spiders is the so-called black widow spider that includes several species of the genus *Latrodectus,* particularly the species *L. mactans mactans* and *L. mactans tredecimguttatus*, which have been found in Eurasia, North America, South America and Africa (Maretic and Stanic, 1954). There is an annual average of 186,000 incidents of poisoning from venomous animals in Mexico, 3,812 of which correspond to spider bites and that have the greatest incidence in the States of Jalisco (786) and Chihuahua (567). From 1992 to 1998, 4 deaths were recorded from spider bites among the beneficiaries of the Mexican Social Security Institute (IMSS): one in Nayarit in 1992 and three in 1994 (in Chiapas, Guanajuato and Estado de México).

Venom from the black widow spider causes a massive release of transmitters with a diversity of nerve endings (Grishin, 1998). This venom is composed of a great variety of toxins of which α-Latrotoxin (α-LTX) is the most studied and exhibits a lethal effect in vertebrates, due to the massive induction of the release of neurotransmitters. The other toxins studied are α-Latrocrustatoxin and the α, β, γ, δ and ε-Latroinsectotoxins that have a similar effect but, as their names indicate, in crustaceans or insects. All the toxins of this venom have a high molecular weight and more than 1,000 amino acids (Grishin, 1998).

Kiyatkin et al. (1990) describe a continuous DNA sequence of 5,408 base pairs with an open reading framework of 4,203 bp that potentially code for a protein with 1401 amino acids (α-Latrotoxin) with a molecular mass of 157,826 Da, whose deduced amino acid sequence they also describe.

Envenomation from the bite of a black widow spider is characterized by the effect of α-Latrotoxin on the massive release of neurotransmitters that is reflected in generalized pain 8 hours after the bite. Pain is present more frequently in the abdomen, but it can also be present in the thighs, flanks and chest. There can be sweating, paleness, nausea, vomiting, breathing and heart problems. Death can occur from cardiac collapse (Key, 1981).

In general terms, there are two lines for the treatment/prevention of envenomation from poisonous animals like the black widow spider: passive immunization (with serotherapeutic agents and fabotherapeutics) and active immunization (through vaccines); the former is a therapeutic measure, while the latter is rather a preventive measure.

Both venoms and toxins have been used in the generation of vaccines. However, exposure to most venoms does not result in protective immunity. Moreover, all the attempts to create protective immunity against venoms with vaccines have failed (Russell, 1971). There have been, however, successes creating this type of immunity against individual toxins, including the vaccines against diphtheria (Audibert et al., 1982) and tetanus (Alouf, 1985).

### A. Active Immunization

Active immunization consists in stimulating the organism itself (the host) that is to be protected from a future envenomation, and the development of antibodies against the venom or toxin (antigen) through its direct application, in such a way that an immunological memory is generated.

One example of the development of a vaccine against a toxin is the tetanus vaccine. Injections of tetanus toxoide provide effective protection, since they obtain a low level of circulation of antibodies and establish an immunological memory. Subsequently, when the immunized organism is exposed to a low dose of the microorganism or tetanus toxin, it can neutralize the microorganism or toxin before further development of the infection or intoxication takes place.

In the case of animal venoms, the generation of a protective immune response is complicated because said venoms are either very difficult to obtain or are very expensive. Furthermore, they normally need to be detoxified and it is extremely difficult to reach the necessary circulating antibody titers to neutralize the large amount of antigen supplied by the poisonous animal, although this is not the case with arachnid venoms that inject little venom. Even when successful immunization has been attained, the immunological memory is usually very slow to respond to the immediate crisis of envenomation.

### B. Passive Immunization

Due to the problems with active immunization, the only treatment available for envenomation is passive immunization. As is the case with active immunization, passive immunization involves the binding of antibodies to antigens. For our purposes, the term antitoxin refers to antibodies (or antibody fragments) raised against individual toxins, while the term antivenom refers to antibodies (or antibody fragments) raised against whole venoms.

In the case of passive immunization, the antibodies that will bind to the venom (antigen) are not produced by the organism that was affected by the envenomation. Generally, an immune response is generated in a first animal. The serum of the first animal is then administered to the individual affected by the envenomation (the host) to provide that individual with a source of specific and reactive antibodies. The antibodies supplied will work then in a certain way as if they were endogenous antibodies, binding the venom toxins and reducing (neutralizing) their toxicity.

The commercial raising of antivenoms can be conducted in different mammals such as mice, rabbits, goats, cows and horses, depending on their end use. The horse is the animal chosen by most laboratories, since it is robust and tolerant to the immunization process and above all because it produces high yields (up to 10 L per bleeding).

However, there are some technical disadvantages in using horses for the production of antivenoms or antitoxins, including the need of large quantities of venom (immunogen or antigen) to perform immunization, obliging the laboratory to have large areas with snake or spider houses in order to have sufficient amounts of the venom.
One of the main producers of antivenoms against snake and arachnid (scorpion and black widow spider) venoms in Mexico and Latin America is Instituto Bioclón S.A. de C.V. which produces antibodies in horses to then purify and hydrolyze them in such a way that their antivenoms are really F(ab)₂ antibody fragments, that is they are fabotherapeutic agents. In particular, they produce an antivenom against the venom of the black widow spider, Aracmyn®, from immunizing horses with the whole spider venom, following the same steps mentioned until the antiserum is obtained that is used to produce the F(ab)₂ fragments that constitute the fabotherapeutic agent.

Some experts in the state of the technique consider that the use of F(ab) and Fab2 fragments, from antibody hydrolysis, in the treatment of envenomations, is outside the concept of serotherapy, given that said fragments no longer constitute serum protein and they call this treatment fabotherapeutics; hence, said antivenom or antitoxin is known as a fabotherapeutic agent rather than a serotherapeutic one. Nevertheless, some other experts in the state of the technique consider that said fragments are still of serum origin and consequently consider them to be serotherapeutic agents. In this invention, we shall use the term "serotherapeutic agent(s)" to refer to the complete antibodies raised against some venom or toxin, and the term "fabotherapeutic agent(s)" to refer to the F(ab) and Fab2 fragments produced by the hydrolysis of antibodies raised against venoms or toxins.

One of the difficult aspects in the clinical handling of envenomation is the lack of standardization of anti-venoms. The recommended doses of therapeutic antivenoms derived from horses are usually given in units of volume. For example, treatment is usually measured by the number of vials to be administered, each vial representing a fixed volume. The potency of individual batches of antivenoms varies due to two main factors: first, because whole sera or immunoglobulin fractions are being used, and because the titer per unit of volume varies from animal to animal and from one day to another, the venom-reactive quantity of antibodies will vary from preparation to preparation. Second, the purification procedures, such as precipitation with ammonium sulfate, can reduce the yield of the active antibody, causing variations in the titer of the active ingredient.

Due to the variety of common and serious side effects of non-purified antivenoms, the doctor must take great care in order to avoid excessive amounts of horse products.

One generally accepted theory is that the high incidence of side effects with present horse antivenoms is due to an excess of irrelevant protein in the antivenom (irrelevant in the sense that they do not have a specific activity against the venom). According to this theory, the removal of said irrelevant protein could reduce the load of the exogenous protein applied to the organism and, consequently, reduce the incidence of adverse immune responses.

Some researchers in the state of the technique have considered the possibility of purification through immunoaffinity. Most of these studies have only examined antibodies against just a single toxin. Yang (1977), for example, tested the purification of antibodies through immunoaffinity against the toxin of a snake's venom. This researcher used cobratoxin, a neurotoxic crystalline protein isolated from the venom of the Taiwan cobra (*Naja naja atra*), bonded to sepharose, as antigen matrix using formic acid to elute the toxin-specific antibodies. It was reported that the antibodies so purified had a greater capacity to neutralize the toxin than the non-purified serum.

Other researchers have followed similar purification schemes such as Kukongviriyapan et al., (1982) who used toxin 3 from the *Naja naja siamensis* linked to different materials to form antigen matrices, thus achieving separation of the horse specific antibodies. Ayeb and Delori (1984) also followed Yang's scheme to purify antibodies against specific scorpion toxins as did Lomonte et al. (1985), who purified antibodies against the *B. asper* myotoxin linked to sepharose.

### BRIEF DESCRIPTION OF THE FIGURES

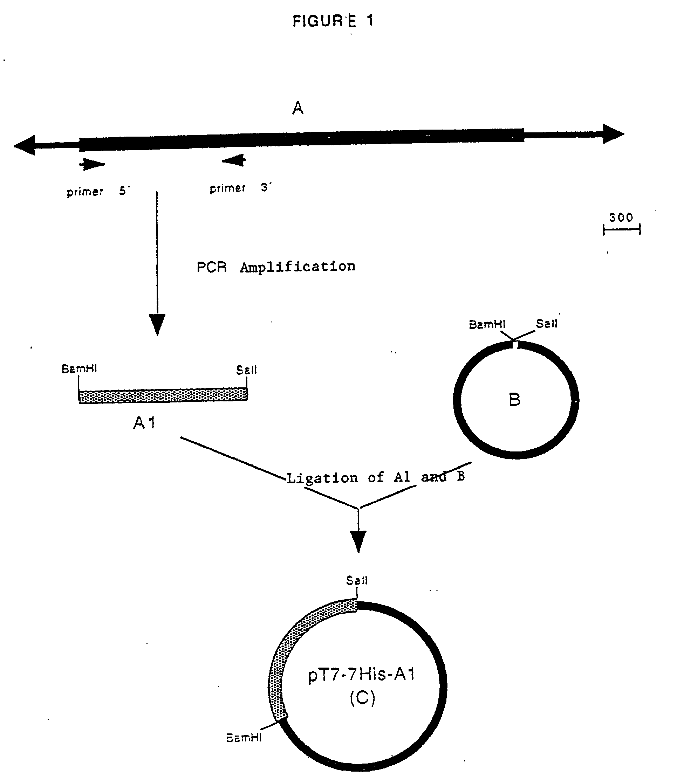

Figure 1. This figure shows the preferred way for the generation of the Expression Construction to produce the recombinant polypeptide of the present invention. The scale is of base pairs. **A** is the open reading coding region of the cDNA of α-Latrotoxin. The 5' and 3' primers were designed to fasten into the ends of the sequence coding the α-Latrotoxin amino acids 1-456 and to insert BamHI and Sail restriction sites. **A1** is the amplification through PCR of the cDNA fragment of α-Latrotoxin between base pairs 142 and 1509 (pT-4N) and then digested with BamHl and Sall. **B** is plasmid pT7-7His digested with the same enzymes. **C** is the structure of the expression construction pT7-7His-A1 obtained by binding **A1** and **B**.

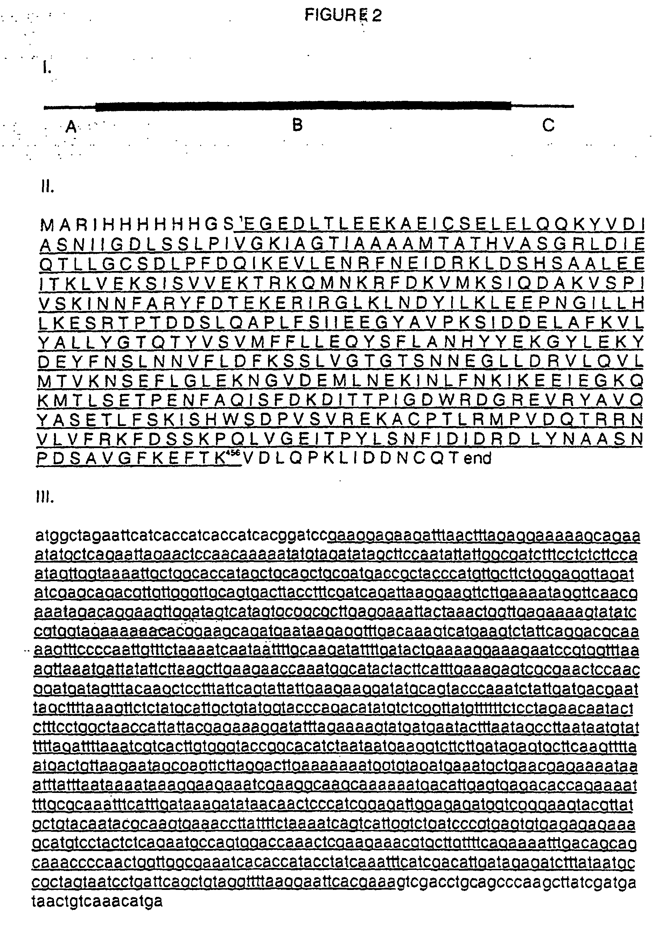

Figure 2. This figure shows, in insert I, a prototype of the recombinant polypeptide of the present invention, where **A** and **C** are fragments derived from the expression vector used, while **B** represents a fragment of the N-terminal region of α-Latrotoxin. Insert 2 shows the amino acid sequence deduced from a preferred example of said recombinant polypeptide where **B** is a fragment of 456 amino acid residues (underlined) of the N-terminal region of α-Latrotoxin. Insert III shows the nucleotide sequence of said preferred recombinant polypeptide where the underlined region is the coding sequence of the fragment of 456 amino acid residues of the N-terminal region of α-Latrotoxin.

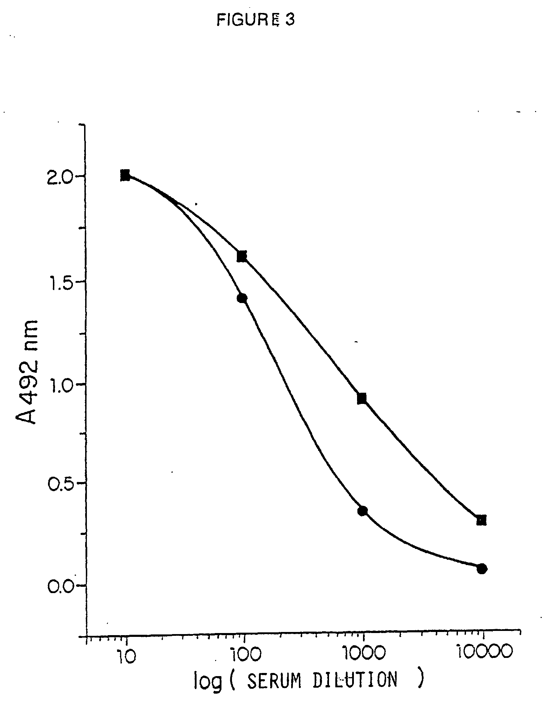

Figure 3. This figure shows the comparative results of binding of a commercial antivenom (raised against the whole venom of the black widow) to the recombinant polypeptide of this invention (●), in comparison with of binding it to the whole venom of the black widow (■).

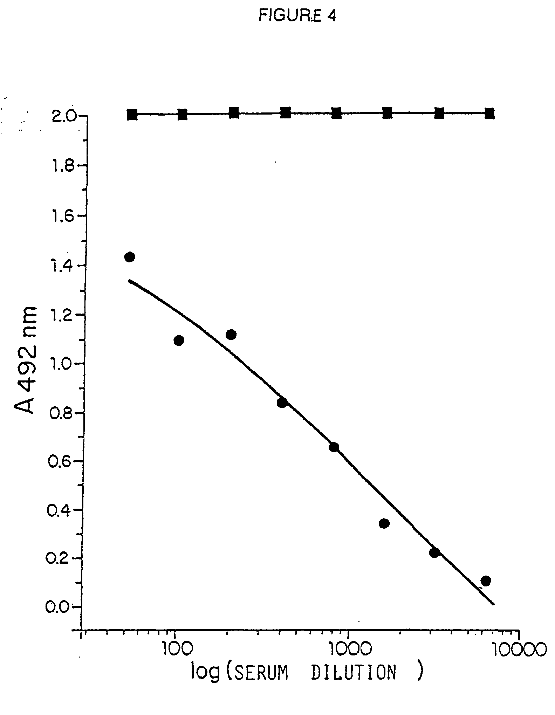

Figure 4. This figure shows the comparative results of bonding of the serum of this invention (raised against the recombinant polypeptide of this invention) to the recombinant polypeptide of this invention (■) and to the whole venom of the black widow (●).

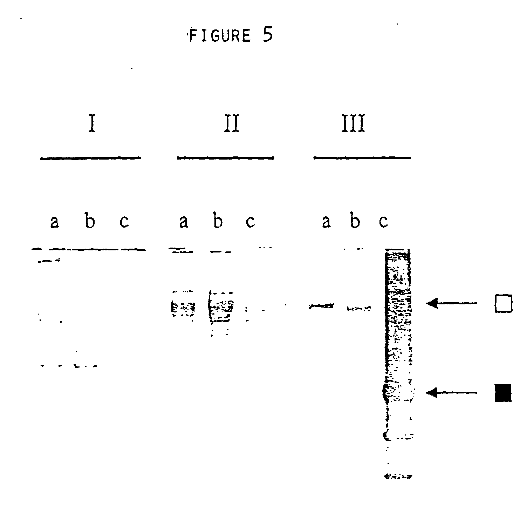

Figure 5. This figure shows the results of the immunoblot in which reactivity of the serum of this invention was tested against the venom of two varieties of the black widow spider and against the recombinant polypeptide of this invention, in comparison with two commercial antivenoms. The "a" lanes contain venom of Latrodectus mactans tredecimguttatus, the "b" lanes contain the venom of Latrodectus mactans mactans and the "c" lanes contain the raw extract of inclusion bodies of the recombinant polypeptide of the present invention. The "I" membrane was treated with the commercial antivenom Aracmyn of Bioclón S.A. de C.V., the "II" membrane was treated with Merck's commercial antivenom and the "III" membrane was treated with the serum of this invention. The bands marked with correspond to the α-Latrotoxin of the venoms and those marked with ■ correspond to the recombinant polypeptide of this invention.

### DETAILED DESCRIPTION OF THE INVENTION

As can be seen from the background, although there are general active immunization schemes, that is vaccination, to prevent intoxications as in the case of tetanus, there is no vaccine to prevent envenomation from the bite of the black widow spider.

Similarly, although there are already some treatments for envenomation from the bite of the black widow spider (*Latrodectus mactans*), another problem arises because the commercial antivenoms available are obtained by means of immunization with the whole venom of the spider. This implies that the serum produced by the animal has a large amount of antibodies against the many different toxins in the venom, most of which have minimal effects on the envenomation process in mammals and, therefore, the antibodies against them do not contribute much to the treatment of envenomation, but do contribute a large amount of exogenous protein to the organism in question and can give rise to severe side effects and damage. In general, some approaches that have been followed to reduce this amount of protein have been the separation of the immunoglobulin fraction in the serum, eliminating other serum proteins (such as albumin) through precipitation with ammonium or sodium sulfate, with the corresponding decrease in neutralizing activity and hydrolysis with pepsin or trypsin of said fraction in order to release the F(ab) or F(ab)2 fragments that conserve the neutralizing activity. However, the antibody fraction or antibody fragments still have a high proportion of irrelevant protein (as they are fragments of antibodies against whole venom).

This problem could be solved by means of purifying through immunoaffinity if there were an immunogenic matrix that would permit the separation from the immunoglobulin source or the fragments arising from their hydrolysis, of those antibodies or specific fragments responsible for neutralizing whole venom. However, this would still have the disadvantage of showing low antivenom yields per animal immunized, since the main toxin responsible for the evenomation effect (α-Latrotoxin) represents only 3% of the whole venom (Grishin, 1998). Furthermore, this presents another problem: there is no antigenic matrix with which to perform said separation.

In order to solve these problems, after having isolated and characterized the cDNA of α-Latrotoxin and deduced the amino acid sequence of the same (Kiyatkin, et al., 1990), the inventors of this invention have approached the strategy of testing some of the fragments of said toxin with respect to its capacity to generate an effective immune response in the neutralization of the toxin itself and in the whole venom.

In Kiyatkin, et al. 1990, the inventors found a series of clones corresponding to different overlapping fragments of α-Latrotoxin, from which they were able to have the complete sequence of the toxin. After aligning this sequence with that of two other latrotoxins, α and δ latroinsectotoxins, they clearly identified four domains in the 3 latrotoxins, the first of which is the peptide signal that in the case of α-Latrotoxin is 20 amino acids; the second is the N-terminal region; the third consists of ankirin-like repeats which presumably participate in binding to presynaptic components of the membrane, and the fourth domain is the C-terminal region. Greater similitude was found between latrotoxins in the N-terminal region where the most notable characteristic are two hydrophobic segments that are well conserved positionally (Grishin, 1998).

Considering the greater similitude in the N-terminal fragment that covers the first 469 amino acids of the toxin, the inventors decided to evaluate this fragment with respect to its immunogenic activity. In order to have sufficient homogeneous quantities of the polypeptide corresponding to the N-terminal fragment of the toxin, they produced said fragment with recombinant DNA techniques.

In order to generate the expression construction, the clone pT-4N was used (Kiyatkin et. al., 1990). This clone consists of nearly 2,300 bp and has an open reading frame that encodes the amino acids residues - 20 to 745 of the toxin, approximately - using it as template in order to amplify the sequence with a Polymerase Chain Reaction (PCR) starting with base pair 142 (the first of the codon corresponding to amino acid residue No. 1 of α-Latrotoxin), in such a way that the amplified DNA comprises the codons corresponding to the N-terminal region of α-Latrotoxin.

Primers were designed to be added to the beginning and end of the sequence, inserting restriction sites in the 5' and 3' ends, selected according to the sites that have the expression vector to be used in the region in which the sequence is to be inserted. It is obvious to an expert in the state of the technique that the choice of the expression vector will depend on the expression system to be used, in such a way that said vector will permit the expression of the inserted fragment in said expression system.

Subsequently, both the expression vector and the amplified fragment to be inserted were digested with the same restriction enzymes and then insertion was performed giving rise to plasmid pT7-7His. Figure 1 shows the generation of the expression construction.

For the polypeptide expression, bacterial cells, particularly *Escherichia coli* BL21 (DE3) were transformed with the recombinant plasmid pT7-7His. For a large scale expression, a culture of said transformed cells was induced with IPTG 1mM and they were incubated at 37º C. The recombinant protein was mainly produced in inclusion bodies.

In order to check that the recombinant polypeptide obtained comprised the N-terminal of α-Latrotoxin, the fragment used as insert was sequenced. Figure 2 insert III shows the nucleotide sequence for the resulting recombinant polypeptide. If this last sequence is compared with the sequence originally reported for clone pT4N (Kiyatkin et al., 1990), some differences can be observed. On sequencing clone pT4N again, it can clearly be seen that this is not a problem of lack of faithfulness in the PCR amplification but lack of precision in determining the sequence in the work reported in 1990.

In figure 2 insert I, a prototype polypeptide of the recombinant polypeptide of this invention is represented where A and C are fragments of the nucleotide sequence derived from the expression vector and/or the first fragments used in the design of the construction and B is a fragment representing the N-terminal region of α-Latrotoxin. As is shown further on, said polypeptide is responsible for generating the effective immune response in neutralizing the whole venom of the black widow spider. Insert II of the same figure shows the primary sequence deduced from a preferred example of said polypeptide, which in this case consists of 482 amino acid residues, only 1 to 456 of which represent the N-terminal region of α-Latrotoxin (the first 456 amino acid residues of the region in this case) and therefore, as is obvious to an average expert in the state of the technique, the first 12 and the last 14 amino acid residues of the sequence (that correspond to fragments derived from the expression vector or from the primers) can be substituted, eliminated or added to the same (in accordance with the expression construction to be used), without substantially affecting the immunogenic activity of the resulting polypeptide. In a similar way, it is obvious to an average expert in the state of the technique that the sequence corresponding to the N-terminal region of α-Latrotoxin which, in this case, was represented by the first 456 amino acids of the toxin, can, in another PCR amplification procedure or with some other technique known in the state of the technique, be represented either by a fragment that is smaller or larger than the 456 amino acid residues, providing they comprise the antigenic determinants necessary to induce the formation of neutralizing antibodies of the whole venom of the black widow spider, or rather said sequence can be modified (altered) in the polypeptide of the present invention by means of the substitution or elimination of any of its amino acids or the insertion of one or more amino acids. These different representations of the N-terminal region or mutations of the same may or may not modify the immunogenic function, therefore those mutations that conserve said function in the resulting recombinant polypeptide are included within the scope of this invention.

With the object of testing the possible effect of the recombinant polypeptide of the present invention on the immune system of mammals, the inventors tested its capacity to generate an immune response and the capacity of the immune response to neutralize the whole venom of the black widow spider.

To do so, they compared the affinity of a commercial anti-venom raised against the whole venom of the black widow spider, using the recombinant polypeptide of this invention in comparison with its affinity with the whole venom of the black widow. The results are shown in figure 3.

A group of mammals was subsequently challenged by injecting them with the antigen (recombinant polypeptide comprising the N-terminal region of α-Latrotoxin). They found that the animals subjected to the treatment did not present symptoms of envenomation or intoxication, thus demonstrating that the antigen does not have harmful effects on mammals (mice), at least in the doses used (up to 100µg per 20g of body weight).

Additionally, procedures were then conducted to determine *in vitro* affinity of the antibodies generated by the treated animals towards the antigen (the recombinant polypeptide that comprises the N-terminal region of α-Latrotoxin) and towards the whole venom of the black widow spider. The procedures are shown in example 7 and as shown in figure 4, the results were highly encouraging.

In order to determine if the antibodies generated against the recombinant polypeptide of the present invention had a neutralizing capacity against the whole venom of the black widow spider, the inventors proceeded as in example 9 to conduct neutralization *in vitro* in order to then inject mammals with the neutralized mixture. The incubated mixture, whole venom ― antibodies, had no toxic effect whatever on the mammals tested and it is therefore considered that on being injected in mammals, the recombinant polypeptide of the present invention awakes an effective immune response for the neutralization (at least *in vitro*) of the whole venom of the black widow spider.

Moreover, in order to determine if the antibodies generated against the recombinant polypeptide of the present invention had a neutralizing capacity *in vivo* against the whole venom of the black widow spider, the inventors proceeded as in example 10 to inject mammals with the whole venom of the black widow spider, to then inject them with a solution of the antibodies that had been previously generated in another group of mammals against the recombinant polypeptide of the present invention. No toxic effect whatever was observed in the mammals that were tested, and it is therefore considered that the recombinant polypeptide of the present invention when injected in mammals arouses an effective immune response for the neutralization (even *in vivo*) of the whole venom of the black widow spider.

For these reasons, the present invention is aimed at a recombinant polypeptide that comprises a fragment of the N-terminal region of α-Latrotoxin, that when it is injected in mammals it arouses an effective immune response in the neutralization of the whole venom of the black widow spider, where said fragment preferably corresponds to the first 456 amino acid residues of α-Latrotoxin, whose amino acid sequence is SEQ.ID.NO:1.

The present invention is also aimed at the DNA fragment that comprises the nucleotide sequence coding for a fragment of the N-terminal region of α-Latrotoxin, where it codes for the recombinant polypeptide of the present invention, wherein said nucleotide sequence is preferably SEQ.ID.NO:2, and the genetic construction for the expression of said DNA fragment, as well as the bacterial cell, particularly from *Escherichia coli*, that comprises said expression construction and at the method of production of the polypeptide of the present invention as the inclusion bodies described above.

In order to determine if the immune response raised against the recombinant polypeptide of the present invention was capable of neutralizing *in vivo* the whole venom of the black widow spider after active immunization, that is, if it worked as a vaccine, as shown in example 13, mammals were immunized using said polypeptide as antigen and then receive different doses of the whole venom of the black widow spider. All the immunized animals survived without presenting envenomation symptoms, after the application of up to 3.3 times the DL50. Not content with these results, the inventors of the present invention decided to challenge the immunized animals again with 3.3 DL50, 3 months after the first challenge (example 14). Once again, the immunized animals survived without presenting any symptom of envenomation and subsequently the animals were challenged 6 months after the second challenge (example 15), without presenting any toxic effect whatever. This demonstrates that when injected in mammals the recombinant polypeptide of the present invention arouses an immune response that is effective in neutralizing the whole venom of the black widow spider. Its use as a vaccine for mammals, which is effective against envenomation from the whole venom of the black widow spider, has been demonstrated, in particular.

Thus, the present invention also refers to the use of the polypeptide of the present invention as vaccine to prevent envenomation from the venom of the black widow spider and the pharmaceutical preparations of said vaccine.

The vaccine of the present invention comprises the polypeptide of the present invention that, in turn, comprises a fragment of the N-terminal region of α-Latrotoxin, preferably obtained by the recombinant method described above. This polypeptide comprises the antigenic determinants necessary to induce the formation of neutralizing antibodies of the whole venom of the black widow spider in the host. Said vaccine is also sufficiently innocuous to be administered without danger of an intoxication, it is stable and compatible with vaccine carriers.

An effective amount of the vaccine must be administered, where "effective amount" is defined as an amount of the polypeptide of the present invention or any functionally equivalent mutation of the same, that is capable of producing an immune response in a mammal. The necessary amount will vary depending on whether the polypeptide of the present invention, or a functionally equivalent mutation of the same is used and the antigenicity of said mutation and on the species and weight of the subject to be vaccinated, but it can be estimated using standard techniques.

Pharmaceutically useful compositions can be formulated as vaccines that comprise the polypeptide of the present invention or any functionally equivalent mutation, in accordance with known methods such as the addition of a pharmaceutically acceptable carrier. In order to form a pharmaceutically acceptable composition suitable for effective administration, said composition shall contain an effective amount of the polypeptide of the present invention or of any functionally equivalent mutation.

The pharmaceutical compositions of the vaccine of the present invention can include a pharmaceutically acceptable adjuvant such as aluminum or calcium gels, modified muramyl dipeptides, monophosphorylate lipids, liposomes, capsules with delayed release, polyglycolic acids and polyamine acids. The polyglycolic and polyamine acids are also useful for the oral administration of the vaccine. Some examples of useful aluminum gels as adjuvants include precipitated aluminum salts such as aluminum phosphate and hydroxide. Some conservatives such as thimerosal, dextran and glycerin can be added to stabilize the final vaccine. If the vaccine is to be in injectable form, immunologically acceptable diluyents or carriers can be included.

The vaccine of the present invention or the pharmaceutical compositions of the same can be administered in mammals, locally and/or systemically, using conventional means such as intravenously, subcutaneously, intramuscularly, intravaginally, intraperitoneally, intranasally, orally or using other mucous routes in order to arouse an effective immune response to protect against the venom of the black widow spider. The vaccine can be administered optionally in sole or multiple doses with the object of sustaining antibody levels.

The pharmaceutical compositions of the vaccine of the present invention must be administered to an individual in such amounts that contain effective amounts of the vaccine of the present invention. The effective amount will vary according to a variety of factors such as the species, condition, weight, sex and age of the individual it will be administered to. Another factor includes the means of administration used.

In another embodiment of the present invention, the recombinant polypeptide that comprises the N-terminal region of α-Latrotoxin is used as immunogen in the immunization of mammals for the generation of antibodies in the serum that are effective in the treatment of other mammals affected by the whole venom of the black widow spider, that is as antivenom. When these antibodies are applied (either in the form of serum or plasma or purified or even as fragments of the same) in an effective quantity in a mammal affected by the venom of the black widow spider, they neutralize the venom *in vivo,* relieving or preventing the advance of the effects of said venom in said organism.

The term "effective amount" applied in the case of passive immunization refers to an amount of antibodies (either as part of the serum or plasma or purified) or their F(ab) or F(ab)2 fragments that, when administered, are capable of preventing or attenuating the effects of envenomation from the venom of the black widow spider. The necessary amount will vary depending on the type and titer of the antibody and the species and weight of the individual to be treated, but it can also be estimated using standard techniques.

The antibodies of the present invention can be obtained by any conventional method known by the experts in the state of the art. In general, an animal (a wide range of animals can be used, of which the most common are mice, rats, rabbits, hamsters, goats, sheep and horses) is immunized with the recombinant polypeptide of the present invention (antigen or immunogen) or with a functionally equivalent mutation of the same in an effective amount, in the absence or presence of an adjuvant or any agent that can increase the effectiveness of the immunogen and optionally the immunization scheme can include reinforcement at regular intervals. Subsequently, the presence of the desired antibodies in the serum of the animal is tested using a suitable method, until the desired titers are obtained. Once collected, the serum or blood of said animal can be used as antibody source (polyclonal) of the present invention.

It is obvious to an average expert in the state of the technique that the serum antibodies that are effective in the neutralization of the whole venom of the black widow spider can be purified by precipitation with ammonium or sodium sulfate by adsorption using a buffer containing at least one polycarboxylic acid as specified in US patent No. 4,933,435, with ethylic alcohol, using an affinity column or any other method available in the state of the technique in order to obtain the corresponding fraction of immunoglobulins, that is, the serotherapeutic agent, a fraction that will show the same function, that is, that will be efficient in the treatment of mammals intoxicated with the venom of the black widow spider, and therefore said serotherapeutical agent will be included within the scope of the present invention.

Similarly, it is obvious to an average expert in the state of the technique that the antibodies of the present invention be they purified, semi-purified or raw (serum) can be hydrolyzed with proteolytic enzymes such as papain, chemopapain, trypsin and chemotrypsin among others, with the object of producing variable F(ab) or F(ab)2 fragments of the same, that conserve the neutralizing activity of the venom of the black widow spider. This can be performed following methods like the one proposed by Landon in US patent No. 5,733,742 in which raw materials such as blood and serum are used to obtain the antibody fragments by enzymatic hydrolysis; or the one proposed by Sullivan in US patent No. 4,849,352 in which a source of antibodies is passed through a papain-polyacrylamide or pepsin-polyacrylamide matrix, thus releasing the corresponding variable fragments; or the method proposed by Ngo in US patent No. 5,328,834 in which the antibodies are fragmented through the use of papain or soluble trypsin and the enzyme is then separated by forming complexes with other specific antibodies against the enzyme. By using such methods, the F(ab) and F(ab)2 fragments conserve the specific activity of the predecessor antibody, therefore the F(ab) or F(ab)2 fragments obtained by hydrolysis of the antibodies of the present invention are included in the scope of the present invention.

The recombinant polypeptide of the present invention can also be used to generate an immunogenic matrix when linked either covalently or through hydrophobic or hydrophilic interactions to some substrate like polyacrylamide, polyvinyl, activated aldehyde agarose (US patents No. 5,904,922 and 5,443,976), sepharose, carboxymethyl cellulose or some other, in such a way that this matrix is capable of specifically binding either antibodies (raised against the toxin α-Latrotoxin, against the whole venom of the black widow spider or against the recombinant polypeptide of the present invention) or the F(ab) or F(ab)2 fragments obtained from the hydrolysis of said antibodies, since it is useful in the purification by immunoaffinity of said antibodies or F(ab) or F(ab)2. Said antigenic matrix is therefore included in the scope of the present invention.

With the purpose of illustrating the scope of the present invention and demonstrating its industrial application, the following examples are given that must in no way be considered as limiting the scope.

### Example 1. Generation of the Expression Construction

In order to generate the expression construction, the inventors particularly decided to use the expression system of *Escherichia coli* and they chose the expression vector pT7-7His. The following primers were designed: AL-1 (5'-TTGGGATCCGAAGGAGAAGATTTAACT-3') y AL-2R (5'-GAACCGTCGACTTTCGTGAATTCCTTAAA-3') that are represented as SEQ.ID.NO:4 and SEQ.ID.NO:6, respectively, in order to insert the restriction sites BamHI and Sall in the 5' and 3' ends respectively, of the amplified fragment, that are found in the expression vector selected. With these primers and using the clone PT-4N as template (Kiyatkin, et. al., 1990), a fragment of the N-terminal region of α-Latrotoxin (between base pair 142 and 1509) was amplified by PCR, which turned out to correspond to the first 456 amino acid residues of α-Latrotoxin. Both the insert and the expression vector pT7-7His were digested with the enzymes BamHI and Sall and the cDNA fragment was inserted in the vector, giving rise to vector pT7-7His-A1.

This construction favors the subsequent purification of the resulting polypeptide by affinity since the addition of a polyhistidine in the amino terminal end makes it join to metals like nickel and a column with metals can be used for said purification.

The nucleotide and amino acid sequence deduced from the resulting polypeptide appear in figure 2, inserts II and III.

### Example 2. Production of the polypeptide using the recombinant method

With the purpose of producing sufficient amounts of the recombinant polypeptide of the present invention, competent cells of *Escherichia coli* BL21 (DE3) were transformed with the vector pT7-7His-A1 obtained as in the example above. The transforming cells were cultivated in a conventional liquid culture medium until their exponential growth and were then induced with IPTG and incubated at 37ºC for 3 hours. The recombinant protein was synthesized principally as insoluble inclusion bodies.

### Example 3. Purification of the Recombinant Polypeptide

A simple method to purify the inclusion bodies used by the inventors of this invention consists of recovering the recombinant cells cultivated as in example 2 and treating them with buffer A (Tris-CI 20mM / Saccharose 20%/EDTA 1 mM, pH 8.0) incubating them for 10 min at room temperature (20 ml of buffer for the cells from 500 ml of culture), recovering them through centrifugation (3,000g, 15 min, 4ºC). The cell pellet is then resuspended in water (20 ml) and incubated for 10 min at room temperature where the cells are recovered through centrifugation in the same conditions. In the following step, the cell pellet is resuspended in 5 ml of buffer P (Tris-Cl 50mM/ NaCl 100mM, pH 8.0) and the cells are lysed through ultrasound. Using this preparation, the inclusion bodies are recovered through centrifugation (10,000g, 20 min, 4ºC), and are subsequently washed several times with buffer W (buffer P added with 25% saccharose) to then be lyophilized until they are used in immunization.

### Example 4. Recognition of the recombinant polypeptide of the present invention by a commercial antivenom raised against the whole venom of the black widow spider.

Recognition of the recombinant polypeptide of the present invention by an antivenom raised against the whole venom of the black widow spider, from Bioclón, was tested using the ELISA method.

As preparation, 96 well polyvinyl trays were recovered with 100 µl/well of a solution with 10 µg/ml of whole venom of the black widow spider in a buffer of phosphates 20 mM pH7.4 with NaCI 0.15 M (PBS) or, with a solution of recombinant polypeptides of the present invention, with an absorbency of 280 mm of 1.2 (the supernatant after dissolving the recombinant polypeptide that still contains inclusion bodies) in the same buffer and were incubated at 4 degrees centigrade over night.

After saturating the unspecific sites with a bovine serum albumin solution (BSA) at 1% 100µl/well of decimal dilutions of the antivenom were added and the mixture was left to react for 4 hours at room temperature. The binding of the antibodies either to the whole venom or the recombinant polypeptide was evidenced using a second antihorse antibody conjugated to peroxidase and revealing the activity of the enzyme with hydrogen peroxide and ortho phenylenediamine as chromogenic substrate. Absorbency at 492 nm was read in an ELISA reader (BIO-RAD model 2550). In figure 3, the absorbency A₄₉₂ graph against the serum dilution is shown.

This demonstrates that the recombinant polypeptide of the present invention is recognized by a commercial anti-venom raised against the whole venom of the black widow spider.

### Example 5. Toxicity of the recombinant polypeptide of the present invention

Twenty female mice were injected with up to 100µg of the recombinant polypeptide of the present invention without any of them showing any intoxication symptoms.

### Example 6. Immunization of mammals with the recombinant polypeptide of the present invention.

As representatives of mammals, mice were chosen due to ease in handling them and experience in doing so. Furthermore, they are model animals universally used for immunological assays.

A group of 20 female mice of the CD1 strain, 3 weeks of age, were immunized under the scheme appearing in table 1.

**Table 1.**

| Mice immunization scheme | | |
|---|---|---|
| Immunization | µg recombinant protein | Adjuvant |
| 1^{st} day 0 | 50 | Complete |
| 2^{nd} day 26 | 50 | Incomplete |
| 3^{rd} day 47 | 100 | Incomplete |
| 4^{th} day 76 | 100 | Incomplete |

Injections were subcutaneous and distributed among 4 points along the animal's back, with a total volume of 100 µl per immunization, using either Freund's complete or incomplete adjuvant.

Approximately 100 µl of blood was obtained from each of the mice on day 61 in order to measure the titer of the antibodies obtained.

A control group with 20 mice was formed with the same characteristics as the experimental group that received the treatment, only the recombinant polypeptide was omitted. At the end of the immunization period, the mice were dry bled, the serum was separated by centrifugation at 3,000 rpm for 10 min, and the supernatant was recovered.

### Example 7. Use of the recombinant polypeptide of the present invention as immunogen in the generation of serum against the whole venom in mammals.

The capacity of the serum obtained from mice immunized with the recombinant polypeptide of the present invention, according to the example above, to recognize the whole venom of the black widow spider using the ELISA method was tested.

Polyvinyl trays (with 96 wells) were re-covered with 100µl/well of a solution with 10µg/ml of whole venom of the black widow spider or 100 µl/well of a solution with 40µg/ml of the recombinant polypeptide of the present invention in PBS. After saturating the unspecific sites with a solution of 1% BSA in PBS, 100 µl per well of double dilutions of mouse antivenom (raised against the recombinant polypeptide of the present invention) were added. The binding of antibodies to the whole venom or recombinant protein was revealed using a second anti-mouse antibody conjugated with peroxidase, using hydrogen peroxide and o-phenylendiamine as chromogenic substrate. Absoreancy at 492 nm was recorded in an ELISA reader.

In figure 4, A₄₉₂ was plotted against the dilution of the serum. A titer of the serum (raised against the recombinant polypeptide of the present invention) of I: 1,258 with respect to the whole venom of the black widow spider was calculated.

This shows that the serum of the present invention is effective in recognizing the whole venom of the black widow spider.

### Example 8. Immunoblot of the whole venom of the black widow spider and the polypeptide against sera raised against the whole venom and against the polypeptide of the present invention.

In order to determine the reactivity of the serum raised against the recombinant polypeptide of the present invention, in comparison with other commercial sera, against the venom of two varieties of black widow spiders and against the recombinant polypeptide, separation was performed by electrophoresis in SDS-PAGE in triplicate (10 to 15 µg of total protein per well), with the whole venom of the spider *Latrodectus mactans mactans* (of Mexican origin) in one lane, the whole venom of the spider *Latrodectus mactans tredecimguttatus*, of Russian origin, in another lane and the raw extract of inclusion bodies of the recombinant polypeptide of the present invention in another lane.

After separation, the separated protein bands of each gel were transmigrated to a nitrocellulose (NC) membrane. The membranes were blocked for 2 hours with skimmed milk and then incubated for another two with a 1:500 dilution of Merck's anti whole venom serum (horse) (one of the membranes), a 1:500 dilution of Bioclón's Aracmyn (anti whole venom horse serum) (another of the membranes) and a serum (mouse) against the recombinant polypeptide of the present invention in the same dilution (the third membrane).

Subsequently, the specific interactions of the horse or mouse antibodies were revealed with the corresponding anti-horse or anti-mouse antibodies, linked to an enzyme and evidenced by the reaction of the enzyme with its substrate, 3,3-Diaminobenzidine (DAB). The results of this immuno-blot are shown in figure 5 where it is clear that the recombinant polypeptide of the present invention is specifically bound by antibodies present in any of the three sera used, while the mouse serum raised against this polypeptide specifically binds components (presumably α-Latrotoxin) of the whole venoms of the two species of the black widow spider.

### Example 9. The use of serum raised against the recombinant polypeptide as neutralizing agent for the whole venom of the black widow spider.

The whole venom of the black widow spider was incubated with the immune serum raised against the recombinant polypeptide of the present invention (with an approximate titer of 1:1,250 with respect to the whole venom of the black widow spider) at 37ºC for one hour. The mixture was injected into mice of the CD1 strain in order to observe the neutralizing capacity of the antivenom. As control, serum was used from mice that had never been immunized.

Groups of mice were injected with the doses of the venom indicated in table 2.

With this, the effectiveness of the serum of the present invention in neutralization, at least *in vitro,* of the black widow spider venom was demonstrated. As the table indicates, experimental groups 1 and 2 were given immune serum, which gave 100% protection to the mice.

**Table 2.**

| Scheme of the protection assay | | | |
|---|---|---|---|
| Group | µg of venom/25g of weight | µl of serum | Survival after 24 h |
| Control | 15 | 50 (non-immune) | 0% |
| Experimental 1 | 15 | 50 (immune) | 100% |
| Experimental 2 | 50 | 50 (immune) | 100% |

### Example 10. Use of the anti polypeptide serum in the passive immunization of others against the whole venom of the black widow spider.

In order to illustrate the capacity of the recombinant polypeptide of the present invention to generate, in mammals, sera efficient in neutralizing the whole venom of the black widow spider, mice were immunized with the polypeptide of the present invention in the same way as in example 6, and blood serum was obtained 6 days later.

Two groups of mice (that had never been in contact with the venom of the black widow spider, with the recombinant polypeptide of the present invention or with sera or antibodies raised against the same) were injected with a 100 lethal dose (enough to kill 100% of the mice in the test), that is 15µg/25g of body weight of the whole venom of the black widow spider. Fifteen minutes later they were given the following treatment: the mice in the experimental groups were injected with 50µl of the serum raised against the recombinant polypeptide of the present invention (with an approximate titer of I: 1,250 with respect to the whole venom of the black widow spider), while the control group was injected with 50µl of a saline solution, obtaining the result shown in table 3.

**Table 3.**

| Results of protection from passive immunization in others | | | |
|---|---|---|---|
| Group | µg of venom/25g of weight | Treatment | Survival after 24h |
| Experimental | 15 | 50 µl of immune serum | 100% |
| Control | 15 | 50µl of saline solution | 0% |

With this it is demonstrated that when the serum of this invention is supplied to another mammal affected by the whole venom of the black widow spider it neutralizes *in vivo* the effect of the venom of said spider, providing effective protection to 100% of the animals challenged.

### Example 11. Purification of the anti polypeptide antibodies of mammal serum.

The antibodies raised against the polypeptide of the present invention can be purified either by physicochemical methods such as adsorption, precipitation with salts or ethylic alcohol or by immunoaffinity when they are passed along a support that either covalently or by hydrophobic or hydrophilic interactions maintains linked some protein with immunoafinity to them as for example the recombinant polypeptide of the present invention as mentioned in example 16. In the first case, there will be partial purification of the corresponding fraction of immunoglobulins that will certainly contain many other inert antibodies of the venom of the black widow spider, while in the second case total purification will be performed that would give as a result antibodies that specifically bind to α-Latrotoxin, the main component of the venom of the black widow spider.

### Example 12. Obtaining a fabotherapeutic agent raised against the recombinant polypeptide of the present invention and its use in the passive immunization of others against the whole venom of the black widow spider.

The antibodies raised against the recombinant polypeptide of this invention produced either in mice, rabbits, goats or preferably in horses can be hydrolyzed with papain or trypsin to produce the corresponding F(ab) or F(ab)2 fragments that conserve the capacity to neutralize the whole venom of the black widow spider.

In order to do so, methods like those proposed in US patents Nos. 5,733,742; 4,849,352 and 5,328,834 can be followed wherein the corresponding variable fragments are obtained through the use of these enzymes.

Blood, serum or some partially purified fraction can be used as an initial antibody source, as in the example above, of the immunoglobulins of the serum raised against the recombinant polypeptide of the present invention.

With the object of reducing the content of exogenous protein supplied with the application of the antivenom, which can cause serious side effects, the serum obtained from immunizing a mammal with the recombinant polypeptide of the present invention can be purified by precipitation with ammonium sulfate. With this, some serum protein fractions that do not intervene in the immune response, like albumin, are eliminated.

### Example 13. The use of the polypeptide as a vaccine against the whole venom of the black widow spider: first challenge.

In order to illustrate the use of the recombinant polypeptide of this invention as vaccine for mammals against the venom of the black widow spider, a direct challenge was conducted in mice that had been previously immunized with the recombinant polypeptide of the present invention through a similar scheme to the one established in example 6, with the whole venom of the black widow spider. This test was performed 17 days after the last immunization.

Four groups of both control mice (without any immunization) and mice that had been previously immunized with the recombinant polypeptide of this invention were injected intraperitoneally with different doses of the whole venom of the black widow spider, obtaining the results shown in table 4.

These results show the feasibility of using the recombinant polypeptide of the present invention as vaccine against the venom of the black widow spider, since 100% of the immunized animals survived.

**Table 4.**

| Results of the use of the recombinant polypeptide as vaccine, 1st challenge | | |
|---|---|---|
| | µg of venom /25 g of weight | Survival after 24 hours |
| Control group | | |
| 1 | 15 | 0% |
| 2 | 15 | 0% |
| 3 | 10 | 80% |
| 4 | 10 | 40% |

| Experimental group | | |
|---|---|---|
| 1 | 15 | 100% |
| 2 | 15 | 100% |
| 3 | 50 | 100% |
| 4 | 50 | 100% |

### Example 14. The use of the polypeptide as vaccine against the whole venom of the black widow spider: second challenge.

In order to confirm the feasibility of using the polypeptide of the present invention as a vaccine against the venom of the black widow spider, a second direct challenge was carried out with mice immunized with the recombinant protein 3 months after the first challenge. The mice that were injected with the whole venom of the black widow spider (50ug/25 g of weight) in the first challenge (groups 3 and 4) were injected again with venom of the black widow spider under the following scheme and with the results shown in table 5.

**Table 5.**

| Results of the use of the recombinant polypeptide as vaccine, 2nd challenge | | |
|---|---|---|
| Group | µg of venom /25 g of weight | Survival after 24 hours |
| Experimental 3 | 15 | 100% |
| Experimental 4 | 50 | 100% |
| Control | 15 | 0% |

The control group were mice of the CD1 strain weighing 33-34 g that had never received any immunization (control for venom potency).

Experimental groups (3 and 4) remained in the animal house in order to continue the studies.

With these results, a further demonstration is given of the feasibility of using the recombinant polypeptide of the present invention as a vaccine against the venom of the black widow spider, since the immunogenic memory or level of neutralizing antibodies remain in the mice, with the capacity to neutralize the venom after a long time (3 months in the mouse's life).

### Example 15. The use of the polypeptide as a vaccine against the whole venom of the black widow spider: third challenge.

In order to confirm the feasibility of using the polypeptide of the present invention as a vaccine against the venom of the black widow spider, a third direct challenge was carried out with mice immunized with the recombinant protein of this invention, 6 months after the second challenge, that is, 9 months after the first one. The mice that were injected with the whole venom of the black widow spider (50ug/25 g of weight) in the first challenge (groups 3 and 4) and with 15 or 50µg in the second challenge, were injected once again with venom of the black widow spider under the following scheme and with the results shown in table 6.

**Table 6.**

| Results of the use of the recombinant polypeptide as vaccine, 3rd challenge | | |
|---|---|---|
| Group | µg of venom /25 g of weight | Survival after 24 hours |
| Experimental 3 | 15 | 100% |
| Experimental 4 | 50 | 100% |
| Control | 15 | 0% |

The control group were mice from the CD1 strain weighing 33-34 g that had never been immunized (control of venom potency).

With these results, a further demonstration is given of the feasibility of using the recombinant polypeptide of the present invention as a vaccine against the venom of the black widow spider, since the immunogenic memory or level of antibodies in circulation with protective capacity still remains in the mice 9 months after the first immunization or 6 months after the last challenge.

### Example 16. Construction of an antigenic matrix through the use of the recombinant polypeptide of the present invention.

With the object of being able to purify through immunoaffinity both the antibodies and their F(ab) or F(ab)2 fragments that neutralize the whole venom of the black widow spider, without it mattering that said antibodies have been raised against the whole venom of the spider or against the recombinant polypeptide of the present invention, said polypeptide can be used in generating an antigenic matrix capable of specifically binding said antibodies or their fragments in such a way that they are separated from the rest of the serum proteins, including antibodies that prove to be inert to the venom of the black widow spider, and that can be subsequently released by passing them through an acid solution, producing a solution of said antibodies or their fragments that have been purified by immunoaffinity.

In order to illustrate the generation of an antigenic matrix, the inventors put the recombinant polypeptide of the present invention dissolved in a buffer of phosphates 20 mM pH7.4 with NaCI 0.15 M (PBS) with an inert polyvinyl surface and incubated it at 4ºC overnight. With this, the recombinant polypeptide was permanently linked by hydrophobic and hydrophilic interactions to the surface of the polyvinyl, forming an antigenic matrix.

In order to determine the capacity of this matrix to specifically bind antibodies raised against the whole venom of the black widow spider or against the recombinant polypeptide of this invention, after saturating the unspecific sites of the polyvinyl with a 1% bovine serum albumin solution (BSA), solutions of different concentrations of sera raised against the whole venom of the black widow spider and against the cited polypeptide were put in contact with the antigenic matrix, left to react at room temperature for 4 hours and then washed to remove all the unspecific serum protein against the whole venom of the black widow spider. The binding of the antibodies to the recombinant polypeptide was evidenced using a second anti-horse antibody conjugated to peroxidase and revealing the activity of the enzyme with hydrogen peroxide and ortho phenylendiamine as chromogenic substrate.

Absorbency was read at 492 nm in an ELISA reader (BIO-RAD model 2550), with which it is shown that the antigenic matrix constructed is capable of specifically binding the antibodies raised against both the recombinant polypeptide of the present invention and those raised against the whole venom of the black widow spider. It is obvious to any average expert in the state of the technique that once the antibodies have been bound in an immunospecific way to the antigenic matrix, they can be recovered with an acid solution like acetic acid or a glycine solution, HCI ph 2.9, and recovered on a buffer solution that neutralizes acidity, reducing the percentage of the protein denatured by the effect of the pH.

### References

Alouf, Ann. Inst. Pasteur/Microbiol. 136B:309 (1985).
Audibert et al., Proc. Natl. Acad. Sci. USA 79:5042 (1982).
Ayeb and Delori, In: Handbook of Natural Toxins, vol. 2, Insect Poisons, Allergens, and Other Invertebrate Venoms (Anthony T. Tu, Ed.) (Marcel Dekker) (1984) Chap. 18, pp. 607-638.
Grishin, E. V. (1998). Black widow spider toxins: the present and the future. Toxicon. 36: 1693-1701
Kiyatkin, N.I., Dulubova, I.E., Chekhovskaya, I.A. and Grishin, E.V. (1990). Cloning and structure of cDNA encoding α-latrotoxin from black widow spider venom. FEBS Lett.. 270:127-131.
Key, G. F. (1981). A comparison of calcium gluconate and methocarbamol in the treatment of latrodectism (Black widow envenomation). Am. J. Trop. Med. Hyg. 30:273-277.
Kukongviriyapan et al., J. Immunol. Meth. 49:97 (1982).
Lomonte et al., Toxicon 23:807 (1985).
Maretic, Z. and Stanic, M. (1954). The health problem of arachnidism. Bull. World. Health Org. 11:1007-1022.
Russell et al., 1985, Preparation Chromatography, Am. J. Trop. Med. Hyg. 34:141-150.
Yang et al., Toxicon 15:51 (1977).
Applications for Mexican patents:
   MX 985,522, Possani L.D., et. al.
US patents:
   US 4,933,435, Ngo; That T
   US 5,733,742, Landon; John
   US 4,849,352, Sullivan; John B. and Russell; Findlay E.
   US 5,328,834, Ngo; That T. and Kumar; Harish P. M
   US 5,904,922, Carroll; Sean B.
   US 5,443,976, Carroll; Sean B.

## Claims

1. A polypeptide that consists of a fragment of the N-terminal region of α-Latrotoxin which, when injected in mammals, is not toxic *per se* and generates an immune response that is effective in neutralizing the whole venom of the black widow spider.

2. The polypeptide of claim 1, wherein the fragment of the N-terminal region of the α-Latrotoxin it comprises has the amino acid sequence SEQ.ID.NO:1, or any mutation which is functionally the equivalent of SEQ.ID.NO:1.

3. A DNA fragment comprising a nucleotide sequence that codes for a fragment of the N-terminal region of α-Latrotoxin, which codes for the polypeptide in claim 1.

4. The DNA fragment of claim 3, wherein the nucleotide sequence it comprises is SEQ.ID.NO:2.

5. A vehicle for the expression in bacteria of the polypeptide of claim 1 or 2 which consists of:
a) A promoter,
b) The DNA fragment of claim 3 or 4 and optionally,
c) A termination sequence.

6. The vehicle of expression of claim 5, wherein said vehicle is the plasmid PT7-7His-A1 preferably.

7. A bacterial cell which contains the expression vehicle of claim 5.

8. The bacterial cell of claim 7 where the bacterium is preferably of the *Escherichia coli* species.

9. A method for the production of the polypeptide of claim 1, which consists of the following, steps:
a) To cultivate the cells of any of claims 7 or 8 in a medium that provides the conditions necessary for the culture and expression of the DNA fragment of claim 3 or 4.
b) To break or lyse the cells obtained and collected in the preceding step, recovering the inclusion bodies released from the broken bacterial cells, and optionally
c) To recover and purify the polypeptide of claim 1 using the inclusion bodies of the preceding step.

10. A polypeptide according to either claim 1 or 2 which is produced by means of the method of claim 9.

11. The use of the polypeptide of any of claims 1, 2 or 10 as vaccine for mammals, effective against envenomation with the venom of the black widow spider.

12. A pharmaceutical composition which comprises the polypeptide of any of claims 1, 2 or 10 and an adequate excipient for administering it subcutaneously, intramuscularly or intranasally, for use in accordance with claim 11.

13. The use of the polypeptide of any of claims 1, 2 or 10 as immunogen for the generation in mammals of sera, effective in the neutralization of the total venom of the black widow spider.

14. A mammal serum obtained according to claim 13 which, when supplied to another mammal affected by the whole venom of the black widow spider, neutralizes *in* *vivo* the effect of the venom of said spider.

15. A serotherapeutic agent obtained by purifying the serum of claim 14 which, when supplied to another mammal affected by the whole venom of the black widow spider, neutralizes *in vivo* the effect of the venom of said spider.

16. A fabotherapeutic agent that comprises F(ab) or F(ab)2 fragments produced by the hydrolysis of the serotherapeutic agent of claim 15 which, when supplied to a mammal that was bitten by a black widow spider, neutralizes *in vivo* the effect of the venom of said spider.

17. An antigenic matrix comprising an inert support that specifically binds to the polypeptide of any of claims 1, 2 and 10 as antigen which, when put into contact with the serotherapeutic agent of claim 15 or the fabotherapeutic agent of claim 16, specifically binds and retains those antibodies or their F(ab) or F(ab)2 fragments that are effective in neutralizing the whole venom of the black widow spider, permitting their purification through immunoaffinity.

18. A serotherapeutic agent, according to claim 15, which is preferably a product of purification through immunoaffinity with the antigenic matrix of claim 17.

19. A fabotherapeutic agent, according to claim 16, which is preferably product of purification through immunoaffinity with the antigenic matrix of claim 17.

20. A pharmaceutical composition which comprises the serum of claim 14, the serotherapeutic agent of any of claims 15 or 18 or the fabotherapeutic agent of any of claims 16 or 19 and an adequate excipient for its intramuscular, intravenous, subcutaneous or intranasal administration.
